## Europäisches Patentamt
## European Patent Office
## Office européen des brevets

(19)

(11) Numéro de publication: **0 169 107**
**B1**

(12) # FASCICULE DE BREVET EUROPÉEN

(45) Date de publication du fascicule du brevet:
**02.09.87**

(51) Int. Cl.⁴: **C 07 D 277/72**

(21) Numéro de dépôt: **85401160.8**

(22) Date de dépôt: **12.06.85**

(54) **Procédé de purification du mercaptobenzothiazole.**

(30) Priorité: **15.06.84 FR 8409448**

(43) Date de publication de la demande:
**22.01.86 Bulletin 86/4**

(45) Mention de la délivrance du brevet:
**02.09.87 Bulletin 87/36**

(84) Etats contractants désignés:
**BE DE FR GB IT LU NL**

(56) Documents cité:
**EP-A-0 015 802**

**CHEMICAL ABSTRACTS, vol. 91, no. 21, 19 novembre 1979, page 664, no. 175331q, Columbus, Ohio, US**

(73) Titulaire: **MANUFACTURE LANDAISE DE PRODUITS CHIMIQUES, F-40370 Rion- des- Landes (FR)**

(72) Inventeur: **Alicot, Michel, Route d'Arreau, F-65250 La Barthe de Neste (FR)**

(74) Mandataire: **Leboulenger, Jean, ATOCHEM Département Propriété Industrielle, F-92091 Paris la Défense 10 Cédex 42 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

Le mercapto-2 benzothiazole est connu dans l'industrie de transformation des élastomères pour son utilisation comme accélérateur de vulcanisation. Il est surtout une matière première importante dans la synthèse d'accélérateurs de vulcanisation plus élaborés permettant à la fois de retarder le temps de grillage des mélanges à vulcaniser et de choisir, pour un objet déterminé à fabriquer, les meilleurs paramètres de vulcanisation. Il est également utilisé dans la synthèse de composés phytosanitaires ou pharmaceutiques et entre aussi comme agent anticorrosion dans les formulations de protection des métaux.

La plupart des procédés de fabrication connus, dérivés fondamentalement du brevet US 1 631 871, font appel à la réaction, à haute température et haute pression, de l'aniline, du soufre et du sulfure de carbone. D'autres font appel, soit à la réaction de la thiocarbanilide, du sulfure de carbone et du soufre (brevet US 1 712 968), soit à la réaction de l'orthochloronitrobenzène, de l'hydrogène sulfuré ou d'un sulfure alcalin et du sulfure de carbone (brevet US 1 960 205), soit encore à la réaction du benzothiazole et du soufre (brevet DE 2 551 060). Le produit de réaction obtenu par ces divers procédés n'est pas utilisable tel quel. Il contient, en effet, des matières premières n'ayant pas réagi (par exemple de l'aniline), des sous-produits et intermédiaires tels que le benzothiazole et l'anilinobenzothiazole. Une purification soigneuse du produit brut de la réaction est nécessaire.

De nombreux procédés de purification ont été proposés à ce jour et font l'objet des brevets US: 1 631 871, 2 090 233, 2 658 864, 2 730 528, 3 030 373, 3 031 073 et 3 818 025 et des brevets FR 2 135 807 et 2 397 409. Pour une analyse de ces procédés on pourra éventuellement se reporter aux pages 2 à 4 du brevet FR 2 450 828 qui expose leurs inconvénients respectifs et propose une autre technique de purification consistant à traiter le produit brut de synthèse par de l'aniline, matière première de la réaction. Selon cette technique, l'aniline peut être ajoutée au produit brut:

. soit directement dans le réacteur de synthèse, avant détente à la pression atmosphérique à une température préférentiellement choisie dans l'intervalle 180-220°C;

. soit, après détente à la pression atmosphérique, à une température comprise entre la température ambiante et la température d'ébullition de l'aniline, c'est-à-dire dans l'intervalle 15-184°C.

Le mercaptobenzothiazole, insolubilisé dans l'aniline, est filtré après refroidissement à la température ambiante et lavé avec de l'aniline. L'élimination de l'aniline contenue dans le gâteau de filtration peut être effectué, par exemple, par entraînement à la vapeur d'eau ou par évaporation sous vide.

En raison de la solubilité du mercaptobenzothiazole dans l'aniline, il est indispensable pour l'économie du procédé, de recycler les phases organiques provenant de la filtration et du lavage du produit cristallisé.

Le procédé de cette technique selon le brevet FR 2 450 828 représente une amélioration très nette par rapport aux procédés antérieurs puisqu'il permet de récupérer sans difficulté les matières premières n'ayant pas réagi et les produits secondaires valorisables pouvant se trouver dans le produit de réaction (benzothiazole en particulier); cette récupération est facilitée par le fait qu'il n'est pas fait appel à d'autres composés que les matières premières de la réaction. Ce procédé supprime pratiquement les problèmes posés pour le traitement des rejets aqueux des installations industrielles classiques.

Cependant, l'inconvénient du procédé selon le brevet FR 2 450 828 réside dans la teneur importante d'aniline qu'il est nécessaire de récupérer du produit isolé à la température ambiante. Cette teneur étant de l'ordre de 40 à 50 %, le bilan thermique de cette récupération, défavorable, grève sérieusement les avantages du procédé.

Il a maintenant été trouvé que l'on peut conserver les avantages d'un procédé de purification par l'aniline et supprimer ses inconvénients. Cette découverte procède des observations suivantes:

1. Lorsque l'on cristallise le mercaptobenzothiazole dans l'aniline, le composé cristallisé obtenu à la température ambiante ou à une température voisine (15 à 30°C) n'est pas le mercaptobenzothiazole lui-même, mais un composé d'addition du mercaptobenzothiazole et de l'aniline. Ce composé est stable dans les conditions de température citées. Par lavage à l'eau (également maintenue à 15-30°C), l'aniline interstitielle est facilement éliminée. Dans le produit cristallisé résultant (en général sous la forme de petits cubes), on dose une teneur en aniline très proche de la teneur théorique (35,77 %) contenue dans le produit d'addition mercaptobenzothiazole aniline.

2. Par contre, lorsque l'on maintient la température de la suspension, obtenu par cristallisation du mercaptobenzothiazole dans l'aniline, à une valeur supérieure à 40°C, on observe que les cristaux du composé précipité ont en général la forme d'aiguilles. Par filtration, à une température supérieure à 40°C et lavage à l'eau, également maintenue à une température supérieure à 40°C, on obtient un composé pratiquement exempt d'aniline et dont les caractéristiques sont celles du mercaptobenzothiazole.

La présente invention a donc pour objet un procédé de purification du mercaptobenzothiazole comprenant les étapes suivantes:

. traitement par l'aniline du produit brut résultant de la synthèse,

. filtration et lavage à l'aniline du mercaptobenzothiazole cristallisé, et

. recyclage éventuel des phases organiques liquides du milieu de purification,

ce procédé étant caractérisé par le fait que la cristallisation du mercaptobenzothiazole, sa filtration et son lavage sont effectués à une température comprise entre 40 et 120°C.

Pour réaliser la purification du mercaptobenzothiazole brut de réaction selon l'invention, il est avantageux d'opérer comme suit:

## 1. Melange de l'aniline et du produit de reaction

L'addition d'aniline peut être réalisée suivant deux variantes:

a) Directement dans le réacteur de synthèse, préférentiellement en fin de réaction, avant détente à la pression atmosphérique. La température est alors comprise entre la température de réaction et la température de solidification du produit brut, soit entre 300°C et 170°C, préférentiellement entre 220°C et 180°C.

b) Par addition de l'aniline au produit brut obtenu après détente à la pression atmosphérique, à une température comprise entre la température ambiante et la température d'ébullition de l'aniline, soit entre 15°C et 184°C, préférentiellement entre 45°C et 120°C. On ne sort pas du cadre de l'invention lorsque le produit brut est ajouté à l'aniline ou lorsque le mélange est effectué par addition simultanée des deux constituants.

## 2. Cristallisation du mercaptobenzothiazole

Quelle que soit, dans les intervalles précédemment définis, la température à laquelle le mélange de l'aniline et du produit brut de réaction est effectué, la cristallisation du mercaptobenzothiazole est faite selon l'invention à une température comprise entre 40 et 120°C, préférentiellement entre 45°C et 80°C.

Ainsi, on peut ajouter le produit brut à l'aniline de manière à maintenir une température de mélange de 100°C et refroidir ensuite à 50°C.

L'addition peut également être faite de telle sorte que la température de mélange soit de 30°C, le mélange étant ensuite réchauffé à 50°C.

## 3. Filtration du mercaptobenzothiazole

Elle est réalisée dans l'intervalle de température choisi pour la cristallisation, soit entre 40°C et 120°C, préférentiellement entre 45°C et 80°C.

## 4. Lavage du mercaptobenzothiazole

Pour éliminer les impuretés, les cristaux filtrés sont lavés à l'aniline, également maintenue à une température comprise entre 40 et 120°C, préférentiellement entre 45°C et 80°C.

## 5. Elimination de l'aniline

Les cristaux de mercaptobenzothiazole purifié sont ensuite lavés avec de l'eau maintenue à une température comprise entre 40°C et 120°C, préférentiellement entre 45°C et 80°C. Entre 100 et 120°C, l'eau est employée sous forme vapeur.

## 6. Sechage du mercaptobenzothiazole

Après lavage à l'eau, les cristaux sont séchés par tout moyen connu, puis broyés pour obtenir la granulométrie requise pour l'application choisie.

La limite inférieure (40°C) de l'intervalle de température défini selon l'invention correspond à la température de dissociation du composé d'addition mercaptobenzothiazole-aniline en ses deux constituants.

La limite supérieure (120°C) est choisie de manière à éviter de solubiliser une trop grande quantité de mercaptobenzothiazole dans les phases organiques issues de la filtration et du lavage à l'aniline.

Il est souhaitable pour l'économie du procédé, de recycler ces phases. Ce recyclage conduit à opérer une purge des impuretés de la boucle de cristallisation. Par élimination d'un volume déterminé de phase organique provenant de la filtration du mercaptobenzothiazole, on sort de la boucle de cristallisation un poids d'impuretés égal à celui qui est apporté par le produit de réaction.

Ceci suppose que, dans une période de mise en régime, le recyclage des phases organiques liquides est effectué un certain nombre de fois sans purge: de la sorte, le taux d'impuretés devenant suffisamment élevé par rapport à celui du mercaptobenzothiazole, les pertes en ce dernier produit deviennent économiquement acceptables.

De la purge peuvent être récupérés en particulier, l'aniline et le benzothiazole.

Bien entendu, on ne sortirait pas du cadre de l'invention en ne recyclant pas les phases liquides ou en ne les recyclant que partiellement.

La quantité pondérale d'aniline à utiliser dans la première étape du procédé selon l'invention, soit sous forme d'aniline pure ou sous forme de phase organique liquide recyclée, peut varier de 2,5 à 6 fois celle du mercaptobenzothiazole brut à purifier et est de préférence comprise entre 3 et 4 fois.

La quantité en poids d'aniline à utiliser pour le lavage peut varier de 0,4 à 1,2 fois celle du mercaptobenzothiazole compté en produit isolé sec et est de préférence comprise entre 0,5 et 0,8 fois.

L'exemple suivant illustre l'invention sans la limiter.

## Exemple

Cet exemple, relatif à la purification du produit brut issu de la réaction du soufre, de l'aniline et du sulfure de carbone, décrit le processus opératoire après la mise en équilibre au cours de laquelle la concentration en impuretes de la phase organique issue de la filtration a atteint une valeur suffisamment élevée (dans cet exemple 35 %).

Dans un appareil muni d'une agitation et d'une prise de température, on introduit 1000 grammes d'aniline recyclée provenant d'une opération précédente. On ajoute 300 grammes de produit de réaction brut à purifier titrant 87 % en mercaptobenzothiazole, prélevés en sortie du réacteur de synthèse après détente à la pression atmosphérique.

Le mélange est effectué de manière à maintenir une température de 90°C. La solution obtenue est ensuite refroidie à 50°C et la suspension obtenue est filtrée dans un filtre thermostatique maintenu à 50°C.

Le produit sur filtre est lavé avec 150 grammes d'aniline ajoutée en trois portions et maintenue à 50°C, puis avec 1000 grammes d'eau ajoutée par portions de 200 grammes et également maintenue à 50°C.

Après essorage et séchage à 100°C, on obtient 255,5 grammes de mercaptobenzothiazole de titre 99 % et de point de fusion (non corrigé) de 178-181°C.

Le rendement de purification est de 96,9 % compté sur le mercaptobenzothiazole 100 %.

Sur le filtrat issu de la filtration de la suspension de mercaptobenzothiazole et refroidi à 20°C, on prélève sur la phase liquide, un poids déterminé permettant d'éliminer une quantité d'impuretés égale à celle entrant dans la boucle de cristallisation par le produit de réaction. L'ensemble des phases organiques sont réunies et recyclées dans une nouvelle opération de purification.

## Revendications

1. Procédé de purification du mercaptobenzothiazole comprenant les étapes suivantes:
. traitement par l'aniline du produit brut de synthèse,
. filtration et lavage à l'aniline du mercaptobenzothiazole cristallisé, et
. recyclage éventuel des phases organiques liquides du milieu de purification, caractérisé en ce que la cristallisation du mercaptobenzothiazole, sa filtration et son lavage sont effectués à une température comprise entre 40 et 120°C.

2. Procédé selon la revendication 1, dans lequel l'aniline est mélangée au produit brut, obtenu après détente à la pression atmosphérique, à une température comprise entre la température ambiante et 184°C, puis on amène la température du mélange à une valeur comprise entre 40 et 120°C.

3. Procédé selon la revendication 1, dans lequel l'aniline est mélangée au produit brut de la réaction, directement dans le réacteur de synthèse entre 170 et 300°C, de préférence entre 180 et 220°C, puis après détente à la pression atmosphérique on refroidit le mélange jusqu'à une température comprise entre 40 et 120°C.

4. Procédé selon l'une des revendications 1 à 3, dans lequel le lavage à l'aniline est suivi d'un lavage à l'eau à une température comprise entre 40 et 120°C.

5. Procédé selon l'une des revendications 1 à 4, dans lequel la cristallisation du mercaptobenzothiazole, sa filtration et son lavage est effectué à une température comprise entre 45 et 80°C, de préférence à environ 50°C.

6. Procédé selon l'une des revendications 1 à 5, dans lequel on recycle les phases organiques liquides.

7. Procédé selon l'une des revendications 1 à 6, dans lequel on purifie un produit brut résultant de la réaction d'aniline, de soufre et de sulfure de carbone.

## Patentansprüche

1. Verfahren zur Reinigung von Mercaptobenzothiazol umfassend die nachfolgenden Schritte:
- Behandlung des rohen Syntheseprodukts mit Anilin,
- Filtration und Waschen des kristallisierten Mercaptobenzothiazols mit Anilin und
- gegebenenfalls Rückführung der flüssigen organischen Phasen des Reinigungsmediums, dadurch gekennzeichnet, daß die Kristallisation des Mercaptobenzothiazols, die Filtration und das Waschen bei einer Temperatur zwischen 40 und 120°C ausgeführt werden.

2. Verfahren nach Anspruch 1, bei dem das Anilin dem nach der Entspannung auf Atmosphärendruck erhaltenen Rohprodukt bei einer Temperatur zwischen Raumtemperatur und 184°C beigemischt wird, und man dann die Temperatur der Mischung auf einen Wert zwischen 40 und 120°C bringt.

3. Verfahren nach Anspruch 1, bei dem das Anilin dem rohen Reaktionsprodukt direkt im Synthesereaktor zwischen 170 und 300°C, vorzugsweise zwischen 180 und 220°C beigemischt wird, und man dann nach Entspannung auf Atmosphärendruck die Mischung bis auf eine Temperatur zwischen 40 und 120°C abkühlt.

4. Verfahren nach einem der Ansprüche 1 bis 3, bei dem auf das Waschen mit Anilin ein Waschvorgang mit Wasser bei einer Temperatur zwischen 40 und 120°C folgt.

5. Verfahren nach einem der Ansprüche 1 bis 4, bei dem die Kristallisation des Mercaptobenzothiazols, die Filtration und das Waschen bei einer Temperatur zwischen 45 und 80°C, vorzugsweise bei ungefähr 50°C durchgeführt werden.

6. Verfahren nach einem der Ansprüche 1 bis 5, bei dem man die flüssigen organischen Phasen zurückführt.

7. Verfahren nach einem der Ansprüche 1 bis 6, bei dem man ein aus der Reaktion von Anilin, Schwefel und Schwefelkohlenstoff erhaltenes Rohprodukt reinigt.

**Claims**

1. Procese for the purification of mercaptobenzothiazole comprising the following steps:
   - treatment with aniline of the crude synthesis product,
   - filtration and washing with aniline of the crystallised mercaptobenzothiazole, and
   - optional recycling of the liquid organic phases of the purification medium,
   characterised in that the crystallisation of mercaptobenzothiazole, its filtration and its washing are carried out at a temperature of between 40 and 120°C.

2. Process according to Claim 1, in which aniline is mixed with the crude product obtained after the pressure has been reduced to atmospheric, at a temperature between ambient temperature and 184°C, and then the temperature of the mixture is brought to a value of between 40 and 120°C.

3. Process according to Claim 1, in which aniline is mixed with the crude reaction product, directly in the synthesis reactor between 170 and 300°C, preferably between 180 and 220°C, and then, after the pressure has been reduced to atmospheric, the mixture is cooled to a temperature of between 40 and 120°C.

4. Process according to one of Claims 1 to 3, in which washing with aniline is followed by washing with water at a temperature of between 40 and 120°C.

5. Process according to one of Claims 1 to 4, in which crystallisation of mercaptobenzothiazole, its filtration and its washing are carried out at a temperature of between 45 and 80°C, preferably at approximately 50°C.

6. Process according to one of Claims 1 to 5, in which the liquid organic phases are recycled.

7. Process according to one of Claims 1 to 6, in which a crude product resulting from the reaction of aniline, sulphur and carbon sulphide is purified.